# EUROPEAN PATENT SPECIFICATION

(11) **EP 1 981 915 B1**
(45) Date of publication and mention of the grant of the patent: **25.04.2018**
(21) Application number: 07703313.2
(22) Date of filing: 06.02.2007
(51) Int. Cl.: A23L 2/58, A61K 8/31, C08L 3/06

(54) **COMPOSITIONS OF ACTIVE INGREDIENTS**
ZUSAMMENSETZUNGEN AUS WIRKSTOFFEN
COMPOSITIONS D'INGREDIENTS ACTIFS

(30) Priority: 06.02.2006 EP 06002418; 06.02.2006 EP 06002419; 06.02.2006 EP 06002421; 06.02.2006 EP 06002420
(43) Date of publication of application: 22.10.2008
(73) Proprietor: DSM IP Assets B.V., 6411 TE Heerlen (NL)
(72) Inventor: SCHAFFNER, David, 4310 Rheinfelden (CH); SCHÄFER, Christian, 79618 Rheinfelden (DE); SCHLEGEL, Bernd, 79618 Rheinfelden (DE)
(74) Representative: Kuhn, Dieter
(86) International application number: PCT/EP2007/001015
(87) International publication number: WO 2007/090614

(56) References cited:
- EP-A1- 1 287 748
- WO-A-93/21785
- US-A- 4 761 186
- US-A- 5 185 176
- US-A- 5 756 721
- US-A- 6 162 474

## Description

The present invention relates to improved OSA-starches, a process for the manufacture thereof, as well as to compositions containing active ingredients, preferably fat-soluble active ingredients, and/or colorants in a matrix based on these improved OSA-starches and to a process for preparing these compositions.

The present invention further relates to the use of the compositions of this invention for the enrichment, fortification and/or for the coloration of food, beverages, animal feed, cosmetics or pharmaceutical compositions.

More particularly, the present invention relates to compositions comprising an improved OSA-starch and a fat soluble active ingredient and/or a colorant, especially a carotenoid, to a process for preparing these compositions and the use of these compositions as additives for the enrichment, fortification and/or for the coloration of food, beverages (preferred), animal feed, cosmetics or pharmaceutical compositions; and to food, beverages (preferred), animal feed, cosmetics or pharmaceutical compositions containing such compositions.

If modified polysaccharides as known in the prior art are used as matrix for compositions containing (fat-soluble) active ingredients and/or colorants, the physical parameters of such obtained compositions often differ due to quality differences in the modified polysaccharides. Therefore, a need exists for compositions, wherein the quality of the modified polysaccharide is standardized or even improved.

This need is fulfilled by compositions comprising
i) at least an improved OSA-starch, preferably obtainable by the process of the present invention as described below,
ii) at least a fat-soluble active ingredient, and/or a colorant, and
iii) optionally at least an adjuvant and/or an excipient.

Such compositions are used for the enrichment, fortification and/or coloration of food, beverage, animal feed, cosmetics or pharmaceutical compositions; said use being a further aspect of the invention. Moreover, the invention is related to food, beverage, animal feed, cosmetics or pharmaceutical compositions containing such compositions.

The components i) to iii) are in more detail described in the following.

### Component i)

At least one OSA-starch is preferably used to make a composition of this invention, but it is possible to use a mixture of two or more different OSA-starches in one composition.

Starches are hydrophilic and therefore do not have emulsifying capacities. However, modified starches are made from starches substituted by known chemical methods with hydrophobic moieties. For example starch may be treated with cyclic dicarboxylic acid anhydrides such as succinic anhydrides, substituted with a hydrocarbon chain (see O. B. Wurzburg (editor), "Modified Starches: Properties and Uses, CRC Press, Inc. Boca Raton, Florida, 1986 (and subsequent editions). A particularly preferred modified starch of this invention has the following formula (I) 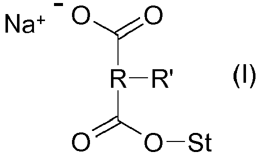 wherein St is a starch, R is an alkylene radical and R' is a hydrophobic group. Preferably R is a lower alkylene radical such as dimethylene or trimethylene. R' may be an alkyl or alkenyl group, preferably having 5 to 18 carbon atoms. A preferred compound of formula (I) is an "OSA-starch" (starch sodium octenyl succinate). The degree of substitution, i.e. the number of esterified hydroxyl groups to the number of free non-esterified hydroxyl groups usually varies in a range of from 0.1% to 10%, preferably in a range of from 0.5% to 4%, more preferably in a range of from 3% to 4%.

The term "OSA-starch" denotes any starch (from any natural source such as corn, waxy maize, waxy corn, wheat, tapioca and potatoe or synthesized) that was treated with octenyl succinic anhydride (OSA). The degree of substitution, i.e. the number of hydroxyl groups esterified with OSA to the number of free non-esterified hydroxyl groups usually varies in a range of from 0.1% to 10%, preferably in a range of from 0.5% to 4%, more preferably in a range of from 3% to 4%. OSA-starches are also known under the expression "modified food starch".

These OSA-starches may contain further hydrocolloids, such as starch, maltodextrin, carbohydrates, gum, corn syrup etc. and optionally any typical emulsifier (as co-emulgator), such as mono- and diglycerides of fatty acids, polyglycerol esters of fatty acids, lecithins, sorbitan monostearate, and plant fibre or sugar.

The term "OSA-starches" encompasses also such starches that are commercially available e.g. from National Starch under the tradenames HiCap 100, Capsul, Capsul HS, Purity Gum 2000, UNI-PURE, HYLON VII; from Roquette Frères ; from CereStar under the tradename C*EmCap or from Tate & Lyle. These commercially available starches are also suitable starting materials for the improved OSA-starches of the present invention.

The term "OSA-starches" encompass further also OSA-starches that were partly hydrolysed enzymatically, e.g. by glycosylases (EC 3.2; see http://www.chem.qmul.ac.uk/iubmb/enzyme/EC3.2/) or hydrolases, as well as to OSA-starches that were partly hydrolysed chemically by know methods. The term "OSA-starches" encompass also OSA-starches that were first partly hydrolysed enzymatically and afterwards additionally hydrolysed chemically. Alternatively it may also be possible to first hydrolyse starch (either enzymatically or chemically or both) and then to treat this partly hydrolysed starch with cyclic dicarboxylic acid anhydrides such as succinic anhydrides, substituted with a hydrocarbon chain, preferably to treat it with octenyl succinic anhydride.

The enzymatical hydrolysis is conventionally carried out at a temperature of from about 5 to about < 100°C, preferably at a temperature of from about 5 to about 70°C, more preferably at a temperature of from about 20 to about 55°C.

The glycosylases/hydrolases can be from fruit, animal origin, bacteria or fungi. The glycolase/hydrolase may have endo-activity and/or exo-activity. Therefore, enzyme preparations of endo- and exo-glycosylases/-hydrolases or any of their mixtures may be used. Usually the glycosylases/hydrolases show also unknown side activities, but which are not critical for the manufacture of the desired product.

Examples of glycosylases are the commercially available enzyme preparations from the suppliers Novozymes, Genencor, AB-Enzymes, DSM Food Specialities, Amano, etc.

Preferably the hydrolases are α-amylases, glucoamylases, β-amylases or debranching enzymes such as isoamylases and pullulanases.

The glycosylase/hydrolase is added to provide a concentration of from about 0.01 to about 10 weight-%, preferably of from about 0.1 to about 1 weight-%, based on the dry weight of the OSA-starch. In a preferred embodiment of the process of the invention, the enzyme is added at once. The enzymatic hydrolysis may also be carried out stepwise. For instance, the glycosylase/hydrolase or a mixture of glycosylases/hydrolases is added to the incubation batch in an amount of e.g. 1 % whereupon, e.g. after 5 to 10 minutes (at a temperature of 35°C) further glycosylase/hydrolase or a mixture of glycosylases/hydrolases which may by the same or different from the first added glycosylase/hydrolase or mixture of glycosylases/hydrolases is added, e.g. in an amount of 2% whereupon the incubation batch is hydrolysed at 35°C for 10 minutes. Using this procedure, starting OSA-starches having a degree of hydrolysis of approximately zero can be used.

The duration of hydrolysis may vary between about a few seconds and about 300 minutes. The exact duration of the enzymatic treatment may be determined in an empirical way with respect to the desired properties of the OSA-starch, such as emulsifying stability, emulsifying capacity, droplet size of the emulsion, depending strongly on parameters like enzyme activities, or composition of the substrate. Alternatively it may be determined by measuring the osmolality (W. Dzwokak and S. Ziajka, Journal of food science, 1999, 64 (3) 393-395).

The inactivation of the glycosylase/hydrolase is suitably achieved by heat denaturation, e.g. by heating of the incubation batch to about 80 to 85°C for 5 to 30 minutes, especially for 5 to 10 minutes.

The term "improved OSA starches" refers to OSA starches, where parts have been separated.

In the case of separation by sedimentation (= centrifugation) and/or microfiltration the parts non soluble at atmospheric pressure in water of a temperature in the range of from 1 to < 100°C (e.g. from 1 to 98°C), preferably in the range of from 30 to 75°C, are separated.

In the case of separation by ultrafiltration parts are separated especially at a temperature in the range of from 1 to < 100°C (e.g. from 1 to 98°C). These parts are not separated according to their solubility but according to their nominal molecular weight cut-off which varies preferably in the range of from 150 Da to 500 KDa, more preferably in the range of from 1 kDa to 200 kDa, most preferably in the range of from 10 kDa to 100 kDa. The trans membrane pressure (TMP) during the ultrafiltration lies preferably in the range of from 0.5 to 3 bar, more preferably in the range of from 0.8 to 2 bar, most preferably in the range of from 0.8 to 1 bar. Small particles are separated off; the parts remaining on the membrane are then further used.

In a preferred embodiment of the present invention the term "improved OSA-starches" refers to OSA-starches, where the turbidity of 10% aqueous solutions of said OSA-starches is in the range of from 1-200 NTU, preferably in the range of from 1-150 NTU, more preferably in the range of from 1-110 NTU, even more preferably in the range of from 1-100 NTU, most preferably ≤ 100 NTU. Such OSA-starches with the given turbidity are also "improved" OSA-starches in the context of the present invention and may be obtained by separating parts non soluble at atmospheric pressure in water of a temperature in the range of from 1 to < 100°C (e.g. from 1 to 98°C) by centrifugation.

The turbidity of said aqueous solutions is measured spectrophotometrically at a wavelength of 455 nm using a HACH 2100 AN Turbidimeter according to USEPA Method 180.1 at room temperature and at atmospheric pressure. The turbidity is then expressed in nephelometric turbidity units (NTU).

### Component ii)

The term "active ingredients" as used herein encompasses "fat-soluble active ingredients" as well as "water-soluble active ingredients". Preferred are "fat-soluble active ingredients".

The term "fat-soluble active ingredient" as used herein encompasses fat soluble vitamins and functionally related compounds which can be used for enrichment or fortification of food, beverages, animal feed, cosmetics or pharmaceutical compositions.

Examples of such fat soluble vitamins are the vitamins of the groups A, D, E or K or derivatives thereof such as their acetates, e.g. vitamin A acetate or tocopherol acetate, or their longer chain fatty acid esters, e.g. vitamin A palmitate or tocopherol palmitate.

Examples of functionally related compounds are e.g. polyunsaturated fatty acids (PUFAs) or derivatives thereof, triglycerides rich in polyunsaturated fatty acids such as eicosapentaenoic acid (EPA), docosahexaenoic acid (DHA) or γ-linolenic acid (GLA), or coenzyme Q 10 (CoQ 10). Also included are fat soluble sun filters, such as UV-A and UV-B filters used in sun care and cosmetic preparations.

The term "colorant" as used herein comprises a carotene or structurally related polyene compound which can be used as a colorant for food, beverages, animal feed, cosmetics or pharmaceutical compositions.

Examples of such carotenes or structurally related polyene compounds are carotenoids such as α-carotene, β-carotene, 8'-apo-β-carotenal, 8'-apo-β-carotenoic acid esters such as the ethyl ester, canthaxanthin, astaxanthin, astaxanthin ester, lycopene, lutein, lutein (di)ester, zeaxanthin or crocetin, α- or β-zeacarotene or mixtures thereof. The preferred carotenoid is β-carotene.

Therefore, a preferred aspect of the invention deals with compositions containing at least an improved OSA-starch and β-carotene as colorant. These compositions, when dissolved, dispersed or diluted in/with water to a final β-carotene concentration of 10 ppm are typically characterised by ultraviolet/visible-spectroscopy using deionised water as reference. At a sample thickness of 1 cm the dispersions show an extinction of at least 0.2 (preferably above 1.0) absorbance units at the wavelength of maximum optical density in the range of 400 to 600 nm. This is equivalent to a formal extinction coefficient of β-carotene in aqueous dispersion E(1%, 1cm) of 200 to 1000 (preferably >1000).

The measuring of E1/1 is explicitly described in example 40.

It is understood that the above named substances of the categories "fat-soluble active ingredient" and "colorant" can also be used as mixtures within the compositions of the present invention.

In a preferred embodiment the amount of the improved OSA starch i) is in the range of from 10 to 99.9 weight-% (preferably in the range of from 20 to 80 weight-%, more preferably in the range of from 40 to 60 weight-%), the amount of the (fat-soluble) active ingredient and/or colorant ii) is in the range of from 0.1 to 90 weight-% (preferably in the range of from 5 to 20 weight-%), and the amount of the adjuvant and/or excipient iii) is in the range of from 0 to 50 weight-%, based on the total amount of the composition.

### Component iii)

Suitably, the compositions of the present invention (further) contain one or more excipients and/or adjuvants selected from the group consisting of monosaccharides, disaccharides, oligosaccharides and polysaccharides, glycerol, triglycerides (different from the triglycerides rich in polyunsaturated fatty acids mentioned above), water-soluble antioxidants and fat-soluble antioxidants.

Examples of mono- and disaccharides which may be present in the compositions of the present invention are sucrose, invert sugar, xylose glucose, fructose, lactose, maltose, saccharose and sugar alcohols.

Examples of the oligo- and polysaccharides are starch, starch hydrolysates, e.g. dextrins and maltodextrins, especially those having the range of 5 to 65 dextrose equivalents (DE), and glucose syrup, especially such having the range of 20 to 95 DE. The term "dextrose equivalent" (DE) denotes the degree of hydrolysis and is a measure of the amount of reducing sugar calculated as D-glucose based on dry weight; the scale is based on native starch having a DE close to 0 and glucose having a DE of 100.

The triglyceride is suitably a vegetable oil or fat, preferably corn oil, sunflower oil, soybean oil, safflower oil, rapeseed oil, peanut oil, palm oil, palm kernel oil, cotton seed oil, olive oil or coconut oil.

Solid compositions may in addition contain an anti-caking agent, such as silicic acid or tricalcium phosphate and the like, and up to 10 weight-%, as a rule 2 to 5 weight-%, of water, based on the total weight of the solid composition.

The water-soluble antioxidant may be for example ascorbic acid or a salt thereof, preferably sodium ascorbate, watersoluble polyphenols such as hydroxytyrosol and oleuropein aglycon, epigallocatechingallate (EGCG) or extracts of rosemary or olives.

The fat-soluble antioxidant may be for example a tocopherol, e.g. dl-α-tocopherol (i.e. synthetic tocopherol), d-α-tocopherol (i.e. natural tocopherol), β- or γ-tocopherol, or a mixture of two or more of these; butylated hydroxytoluene (BHT); butylated hydroxyanisole (BHA); ethoxyquin, propyl gallate; tert. butyl hydroxyquinoline; or 6-ethoxy-1,2-dihydroxy-2,2,4-trimethylquinoline (EMQ), or an ascorbic acid ester of a fatty acid, preferably ascorbyl palmitate or stearate.

Depending on the pH of the aqueous matrix solution the ascorbic acid ester of a fatty acid, particularly ascorbyl palmitate or stearate, may alternatively be added to the water phase.

The compositions of the present invention may be solid compositions, i.e. stable, water-soluble or -dispersible powders, or they may be liquid compositions, i.e. aqueous colloidal solutions or oil-in-water dispersions of the aforementioned powders. The stabilised oil-in-water dispersions, which may be oil-in-water emulsions or may feature a mixture of suspended, i.e. solid, particles and emulsified, i.e. liquid, droplets, may be prepared by the methods described below or by an analogous manner.

More specifically, the present invention is concerned with stable compositions in powder form comprising one or more (fat-soluble) active ingredients and/or one or more colorants in a matrix of an improved OSA starch composition.

Typically, a powder composition according to the present invention comprises

| **Ingredient** | **Amount** |
|---|---|
| an improved OSA starch | 10 to 99.9 weight-%, preferably 20 to 80 weight-%, more preferably 40 to 60 weight-% |
| if the fat-soluble active ingredient is a carotenoid such as β-carotene the right-handed amounts apply | 10 to 99.9 weight-%, preferably 20 to 80 weight-%, more preferably 50 to 70 weight-% |
| a fat soluble active ingredient and/or a colorant | 0.1 to 90 weight-%, preferably 0.5 to 60 weight-% |
| if the fat-soluble active ingredient is a carotenoid such as β-carotene the right-handed amounts apply | 0.01 to 50 weight-%, preferably 0.1 to 50 weight-%, more preferably 0.5 to 30 weight-% |
| a mono- or disaccharide | 0 to 70 weight-%, preferably 0 to 40 weight-% |
| a starch hydrolysate | 0 to 70 weight-%, preferably 0 to 40 weight-% |
| glycerol | 0 to 20 weight-%, preferably 0 to 10 weight-% |
| a triglyceride | 0 to 50 weight-%, preferably 0 to 30 weight-% |
| one or more water-soluble antioxidant(s) | 0 to 5 weight-%, preferably 0 to 2 weight-% |
| one or more fat-soluble antioxidant(s) | 0 to 7 weight-%, 0 to 5 weight-%, preferably 0 to 2 weight-% |
| a starch | 0 to 50 weight-%, preferably 0 to 35 weight-% |
| anti-caking agent | 0 to 5 weight-%, preferably 1 weight-%, preferably 0.5 to 2 weight-% |
| water | 0 to 10 weight-%, preferably 1 to 5 weight-% |

In still another aspect of the invention, the compositions according to the invention may additionally contain proteins (of plant or animal origin) or hydrolysed proteins that act as protective colloids, e.g. proteins from soy, rice (endosperm) or lupin, or hydrolysed proteins from soy, rice (endosperm) or lupin, as well as plant gums (such as Gum Acacia or Gum arabic) or modified plant gums. Such additional proteins or plant gums may be present in the formulations of the invention in an amount of from 1 to 50 weight-% based on the total amount of the improved OSA starch in the formulation/composition.

### Manufacture of component i), the improved OSA starch

The improved OSA-starch can be manufactured by a process comprising the following steps:
a) preparing an aqueous solution or suspension of an OSA-starch, preferably having a dry mass content in the range of from 0.5 to 80 weight-%, based on the total weight of the aqueous solution or suspension, whereby the temperature of the water is preferably in the range of from 1 to < 100°C;
b) separating parts of the OSA-starch, preferably at atmospheric pressure in water of a temperature in the range of from 1 to < 100°C;

In case of separation by sedimentation (centrifugation) or microfiltration the parts to be separated are especially those parts that are not soluble at atmospheric pressure in water of a temperature in the range of from 1 to < 100°C.
c) optionally converting the thus obtained improved OSA-starch into a solid form.

Details of this process are discussed in the following.

### Step a)

In step a) preferably an aqueous solution or suspension of an OSA starch (with the definition and the preferences as described above under the chapter component i)) having a dry mass content in the range of from 0.1 to 80 weight-%, preferably in the range of from 0.5 to 80 weight-%, is prepared when step b) is performed by sedimentation/centrifugation and microfiltration.

It is also possible to use mixtures of OSA-starches. The weight-ratios of a mixture of two different OSA-starches may vary in a range of from 1 : 99 to 99 : 1. Preferably a mixture of HiCap 100 and Capsul HS is used. More preferably a mixture of 50 to 80 weight-% of HiCap 100 and 20 to 50 weight-% of Capsul HS is used. Most preferably a mixture of 50 weight-% of HiCap 100 and 50 weight-% of Capsul HS is used.

In a further preferred embodiment of the invention the water has a temperature in the range of from 30 to 75°C.

### Step b)

Step b) is preferably carried out at a temperature in the range of from 1 to < 100°C (e.g. from 1 to 98°C), more preferably at a temperature in the range of from 30 to 75°C.

Step b) is carried out by sedimentation (preferably by centrifugation) and microfiltration, especially by crossflow microfiltration.

The sedimentation is a method which separates according to the density.

The (micro-)filtration is a method that separates according to the particle size.

If sedimentation/centrifugation and filtration are carried out, usually the sedimentation/centrifugation is first carried out followed by the filtration, i.e. in a preferred embodiment of the present invention a centrifugation is first carried out followed by a microfiltration.

The centrifugation may be carried out at 1000 to 20000 g depending on the dry mass content of the OSA-starch in the aqueous solution or suspension. If the dry mass content of the OSA-starch in the aqueous solution or suspension is high, the applied centrifugation force is also high. For example for an aqueous solution or suspension with a dry mass content of the OSA-starch of 30 weight-% a centrifugation force of 12000 g may be suitable to achieve the desired separation.

The centrifugation may be carried out at dry matter contents in the range of from 0.1-60 weight %, preferably in the range of from 10-50 weight-%, most preferably in the range of from 15-40 weight-% at temperatures in the range of from 2-99°C, preferably in the range of from 10-75°C, most preferably in the range of from 40- 60°C.

Microfiltration in the context of the present invention means that particles that have a size greater than 0.05 µm to 10 µm, especially that particles that have a size greater than 1 µm to 5 µm are separated. These separated parts form the so-called retentate of the microfiltration.

The microfiltration may be performed with hydrophilic membranes such as ceramic membranes (e.g. commercially available from Tami under the name "Ceram inside") or with membranes of regenerated cellulose (e.g. commercially available from Sartorius under the name "Hydrosart") or a porous steel pipe-filter, commercially available from LIGACON W.Röll & CO. AG (Switzerland). These membranes have preferably a pore size in the range of from 0.5 to 5 µm.

In the context of the present invention the parts separated by microfiltration are called the "retentate" whereas the remaining solution without the separated parts is called the "permeate".

The "non-soluble parts" may further be divided in a "solid fraction" and "warm-water soluble parts". The term "warm-water soluble parts" means parts that are not soluble in water of a temperature in the range of from 1 to 30°C, but in water of a temperature of from > 30°C to < 100°C (e.g. from 31 to 98°C).

The term "solid fraction" means parts that are not soluble in water of a temperature in the range of from 1 to < 100°C. Such solid fraction is, thus, even not soluble in water of a temperature in the range of from 30 to < 100°C (e.g. in the range of from 30 to 98°C).

The steps a) and b) may be carried out several times subsequently, and at different temperatures. That means also that if a mixture of two different OSA-starches (e.g. a mixture of HiCap 100 and Capsul) is used, that they may be purified separately or jointly. Surprisingly it has been found out that a mixture of two different OSA-starches, where only one OSA-starch has been improved according to the process of the present invention, even leads to better β-carotene compositions and beverages containing them than the use of a mixture of non-improved OSA-starches. The mixture of two different improved OSA-starches (improved according to the process of the present invention) leads even to better β-carotene compositions and beverages containing them than the use of a mixture of two different OSA-starches, where only one OSA-starch has been improved according to the process of the present invention.

In one embodiment of the present invention the aqueous solution or suspension may be prepared with cold water (water of a temperature of from 1 to 30°C) (step a) and may also be sedimentated (centrifuged) and/or filtered at this temperature (step b).

In another embodiment of the present invention the aqueous solution or suspension may be prepared with warm water (water of a temperature of from > 30 to < 100°C) (step a) and may also be sedimentated (centrifuged) and/or filtered at this temperature (step b).

In a further embodiment of the present invention the aqueous solution or suspension may be prepared with warm water (water of a temperature of from > 30 to < 100°C) (step a), it may then be cooled down to a temperature of below 30°C, and sedimentated (centrifuged) and/or filtered at this lower temperature (step b).

In a further embodiment of the present invention the pH of the aqueous solution or suspension of the OSA-starch is additionally adjusted to a value of from 2 to 5.

### Step c)

The conversion into a solid form, e.g. a dry powder, can be achieved by spray drying or freeze-drying. Spray drying is preferably performed at an inlet temperature of 140°C to 210°C and at an outlet temperature of 50°C to 75°C. The freeze-drying is preferably performed at a temperature of from -20°C to -50°C for 10 to 48 hours.

The solid form may further be granulated.

Especially the process according to the invention for improving the OSA-starch as described above leads to overall improved functional properties of the OSA-starch such as better emulsifying properties, generally higher and faster solubility in aqueous solution as well as better cold water solubility, and better film-forming properties.

A further aspect of the invention is, thus, an improved OSA-starch as obtainable by the process according to the invention described above.

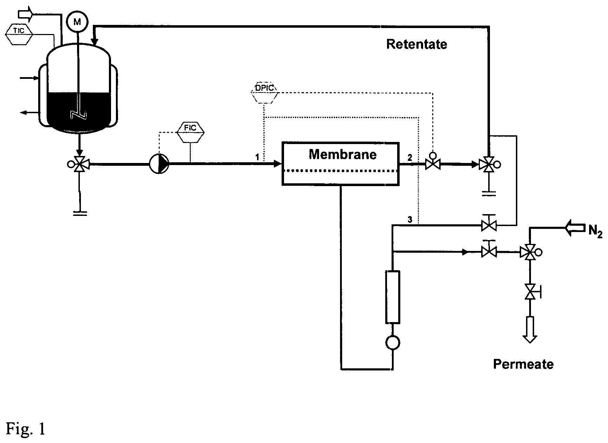

Figure 1 illustrates one embodiment of the present invention whereby the separation is performed by a crossflow microfiltration. Advantageously the membrane is cleaned by a back flow of the permeate in a pulsed mode. Hereby the following abbreviations are used: DP = difference pressure, T = temperature, F = flow, I = indicate, C = control.

### Process for the manufacture of the compositions according to the invention

The present invention is further related to a process for the manufacture of such compositions as described above comprising the following steps:
I) preparing an aqueous solution or colloidal solution of an OSA-starch at a temperature in the range of from 1 to < 100°C,
II) separating parts (in case of separation by centrifugation and/or microfiltration: non-soluble parts) of that aqueous solution or colloidal solution obtained in step I) to obtain an aqueous solution of an improved OSA-starch,
   or instead of performing I and II subsequently carrying out step I-II), i.e.
   preparing an aqueous solution or colloidal solution of an improved OSA-starch, preferably of an improved OSA-starch obtainable by the process of the invention as described above comprising the steps a) to c),
III) optionally adding at least a water-soluble excipient and/or adjuvant to the solution prepared in step I), II) or I-II),
IV) preparing a solution or dispersion of at least an active ingredient, preferably of at least a fat-soluble active ingredient, and/or colorant and optionally at least a fat-soluble adjuvant and/or excipient,
V) mixing the solutions prepared in step II) (or I-II)) to IV) with each other,
VI) homogenising the thus resulting mixture,
VII) optionally converting the dispersion obtained in step VI) into a powder, whereby optionally the parts (in case of centrifugation and/or microfiltration: especially the non-soluble parts) separated in step II) (or step b)) are added partly or completely during or before the conversion, optionally under addition of water, and
VIII) optionally drying the powder obtained in step VII).

This process for the manufacture of the compositions of the present invention can be carried out in an according manner as disclosed for the preparation of matrix-based compositions of (fat-soluble) active ingredient and/or colorant compositions for enrichment, fortification and/or coloration of food, beverages, animal feed, cosmetics or pharmaceutical compositions, e.g. in EP-A 0 285 682, EP-A 0 347 751, EP-A 0 966 889, EP-A 1 066 761, EP-A 1 106 174, WO 98/15195, EP-A 0 937 412, EP-A 0 065 193 or the corresponding US 4,522,743, WO 02/102298, EP-A 1 300 394 and in EP-A 0 347 751, the contents of which are incorporated herein by reference.

Steps I to III encompass the preparation of the matrix, whereby steps V to VI are directed to the preparation of the emulsion.

### Steps I and II

These steps may be carried out as described above for steps a) and b). They may also be carried out subsequently several times. The warm-water soluble parts may as well be separated as the solid fraction as well as both. Mixtures of OSA-starches as already disclosed above for step b) may also be used.

During step I other water-soluble ingredients of the final composition such as water-soluble antioxidants may also be added.

### Step III

Examples of water-soluble excipients and/or adjuvants are monosaccharides, disaccharides, oligosaccharides and polysaccharides, glycerol and water-soluble antioxidants. Examples of them are given above.

Other water-soluble ingredients of the final composition such as water-soluble antioxidants may also be added during step III.

### Step IV

The (fat-soluble) active ingredient and/or colorant and optional fat-soluble excipients and adjuvents are either used as such or dissolved or suspended in a triglyceride and/or an (organic) solvent.

Suitable organic solvents are halogenated aliphatic hydrocarbons, aliphatic ethers, aliphatic and cyclic carbonates, aliphatic esters and cyclic esters (lactones), aliphatic and cyclic ketones, aliphatic alcohols and mixtures thereof.

Examples of halogenated aliphatic hydrocarbons are mono- or polyhalogenated linear, branched or cyclic C₁- to C₁₅-alkanes. Especially preferred examples are mono- or polychlorinated or -brominated linear, branched or cyclic C₁- to C₁₅-alkanes. More preferred are mono- or polychlorinated linear, branched or cyclic C₁- to C₁₅-alkanes. Most preferred are methylene chloride and chloroform.

Examples of aliphatic esters and cyclic esters (lactones) are ethyl acetate, isopropyl acetate and n-butyl acetate; and γ-butyrolactone.

Examples of aliphatic and cyclic ketones are acetone, diethyl ketone and isobutyl methyl ketone; and cyclopentanone and isophorone.

Examples of cyclic carbonates are especially ethylene carbonate and propylene carbonate and mixtures thereof.

Examples of aliphatic ethers are dialkyl ethers, where the alkyl moiety has 1 to 4 carbon atoms. One preferred example is dimethyl ether.

Examples of aliphatic alcohols are ethanol, iso-propanol, propanol and butanol.

Furthermore any oil (triglycerides), orange oil, limonen or the like and water can be used as a solvent.

### Step V

The (fat-soluble) active ingredient and/or colorant or the solution or dispersion thereof, respectively, is then added to the aqueous (colloidal) solution with stirring.

### Step VI

For the homogenisation conventional technologies, such as high-pressure homogenisation, high shear emulsification (rotor-stator systems), micronisation, wet milling, microchanel emulsification, membrane emulsification or ultrasonification can be applied. Other techniques used for the preparation of compositions containing (fat-soluble) active ingredients and/or colorant for enrichment fortification and/or coloration of food, beverages, animal feed, cosmetics or pharmaceutical compositions are disclosed in EP-A 0 937 412 (especially paragraphs [0008], [0014], [0015], [0022] to [0028]), EP-A 1 008 380 (especially paragraphs [0005], [0007], [0008], [0012], [0022], [0023] to [0039]) and in US 6,093,348 (especially column 2, line 24 to column 3, line 32; column 3, line 48 to 65; column 4, line 53 to column 6, line 60), the contents of which are incorporated herein by reference.

### Step VII

The so-obtained dispersion, which is an oil-in-water dispersion, can be converted after removal of the organic solvent (if present) into a solid composition, e.g. a dry powder, using any conventional technology such as spray drying, spray drying in combination with fluidised bed granulation (the latter technique commonly known as fluidised spray drying or FSD), or by a powder-catch technique whereby sprayed emulsion droplets are caught in a bed of an absorbent, such as starch, and subsequently dried.

### Step VIII

Drying may be performed at an inlet-temperature of from 100 to 250°C, preferably of from 150°C to 200°C, more preferably of from 160 to 190°C, and/or at an outlet-temperature of from 45 to 160°C, preferably of from 55 to 110°C, more preferably of from 65 to 95°C.

In case of separation by centrifugation and/or microfiltration "adding the non-soluble parts during the conversion" means that the separated non-soluble parts (either the warm-water soluble parts or the solid fraction or both) may be added after having finalized step VI into the homogenized mixture (the emulsion) or they may be added separately as additional component into the spray-dryer or they may be added to the bed of absorbent or they may be added at several different time points of the process.

In another embodiment of the present invention an OSA-starch (improved according to the present invention or not) or a mixture of two or more different OSA-starches is added to the emulsion before drying.

For the production of liquid and solid product forms such as oil-in-water suspensions, oil-in-water emulsions or powders the improved OSA-starch (as described above) used therein act as multifunctional ingredients.

The present invention is also directed to the use of compositions as described above for the enrichment, fortification and/or coloration of food, beverages, animal feed, cosmetics or pharmaceutical compositions, preferably for the enrichment, fortification and/or coloration of beverages. There is no "ringing", i.e. the undesirable separation of insoluble parts at the surface of bottles filled with beverages containing the compositions of the present invention.

Other aspects of the invention are food, beverages, animal feed, cosmetics and pharmaceutical compositions, especially beverages, containing a composition as described above.

Beverages wherein the product forms of the present invention can be used as a colorant or a functional ingredient can be carbonated beverages e.g., flavoured seltzer waters, soft drinks or mineral drinks, as well as non-carbonated beverages e.g. flavoured waters, fruit juices, fruit punches and concentrated forms of these beverages. They may be based on natural fruit or vegetable juices or on artificial flavours. Also included are alcoholic beverages and instant beverage powders. Besides, sugar containing beverages diet beverages with non-caloric and artificial sweeteners are also included.

Further, dairy products, obtained from natural sources or synthetic, are within the scope of the food products wherein the product forms of the present invention can be used as a colorant or as a functional ingredient. Typical examples of such products are milk drinks, ice cream, cheese, yoghurt and the like. Milk replacing products such as soymilk drinks and tofu products are also comprised within this range of application.

Also included are sweets which contain the product forms of the present invention as a colorant or as a functional ingredient, such as confectionery products, candies, gums, desserts, e.g. ice cream, jellies, puddings, instant pudding powders and the like.

Also included are cereals, snacks, cookies, pasta, soups and sauces, mayonnaise, salad dressings and the like which contain the product forms of the present invention as a colorant or a functional ingredient. Furthermore, fruit preparations used for dairy and cereals are also included.

The final concentration of the (fat-soluble) active ingredient and/or the colorant which is added via the product forms of the present invention to the food products may be from 0.1 to 500 ppm, particularly from 1 to 50 ppm based on the total weight of the food composition and depending on the particular food product to be coloured or fortified and the intended grade of coloration or fortification.

The final concentration of the (fat-soluble) active ingredient and/or the colorant, especially of β-carotene, which is added via the product forms of the present invention to beverages may be from 0.1 to 50 ppm, particularly from 1 to 30 ppm, more preferably from 3 to 20 ppm, based on the total weight of the beverage and depending on the particular beverage to be coloured or fortified and the intended grade of coloration or fortification.

The food compositions of this invention are preferably obtained by adding to a food product the (fat-soluble) active ingredient and/or the colorant in the form of a composition of this invention. For coloration or fortification of a food or a pharmaceutical product a composition of this invention can be used according to methods per se known for the application of water dispersible solid product forms.

In general the composition may be added either as an aqueous stock solution, a dry powder mix or a pre-blend with other suitable food ingredients according to the specific application. Mixing can be done e.g. using a dry powder blender, a low shear mixer, a high-pressure homogeniser or a high shear mixer depending on the formulation of the final application. As will be readily apparent such technicalities are within the skill of the expert.

Pharmaceutical compositions such as tablets or capsules wherein the compositions are used as a colorant are also within the scope of the present invention. The coloration of tablets can be accomplished by adding the product forms in form of a liquid or solid colorant composition separately to the tablet coating mixture or by adding a colorant composition to one of the components of the tablet coating mixture. Coloured hard or soft-shell capsules can be prepared by incorporating a colorant composition in the aqueous solution of the capsule mass.

Pharmaceutical compositions such as tablets such as chewable tablets, effervescent tablets or filmcoated tablets or capsules such as hard shell capsules wherein the compositions are used as an active ingredient are also within the scope of the present invention. The product forms are typically added as powders to the tableting mixture or filled into the capsules in a manner per se known for the production of capsules.

Animal feed products such as premixes of nutritional ingredients, compound feeds, milk replacers, liquid diets or feed preparations wherein the compositions are either used as a colorant for pigmentation e.g. for egg yolks, table poultry, broilers or aquatic animals or as an active ingredient are also within the scope of the present invention.

Cosmetics, toiletries and derma products i.e. skin and hair care products such as creams, lotions, baths, lipsticks, shampoos, conditioners, sprays or gels wherein the compositions are used as a colorant or as an active ingredient are also within the scope of the present invention.

The following non limiting examples illustrate the invention further.

### Examples

### Example 1: Microfiltration of an OSA-starch with a ceramic membrane with a pore size of 1.4 µm.

An aqueous 45 weight-% solution of HiCap 100 (commercially available from National Starch) was filtered through a ceramic membrane with a pore size of 1.4 µm at 50°C. As the analytic result shows the water content of the permeate is more or less unchanged when compared with the water content of the starting solution. Nevertheless the permeate shows a much lower turbidity.

### Example 2: Microfiltration of an OSA-starch with a ceramic membrane with a pore size of 0.2 µm.

An aqueous 35 weight-% solution of HiCap 100 (commercially available from National Starch) was filtered through a ceramic membrane with a pore size of 0.2 µm at 50°C. Here a strong separation by atomic weight and molecular structure appeared. So the permeate had a solid fraction of only 25.9 weight-%. Therefore, water was removed from the permeate to get a final concentration of the solids of 42.5 weight-% to be able to compare the results of the emulsification trials with the permeate of example 1.

The following table 1 shows the weight-% of the solid fraction of the starting solution, the retentate, the permeate and as used in the emulsification trial.

**Table 1**

| **Microfiltration** | Solid fraction [%] | | | |
|---|---|---|---|---|
| Hi-Cap 100 | Solution | Retentate | Permeate | Emulsification trial |
| No physical modification = un-improved Hi-Cap 100 | 44 | - | - | 47.47 |
| Permeate (pore size 1.4 µm) (example 1) | 45 | 44.12 | 42.49 | 42.49 |
| Permeate (pore size 0.2 µm) (example 2) | 35 | 41.17 | 25.94 | 41.82 |

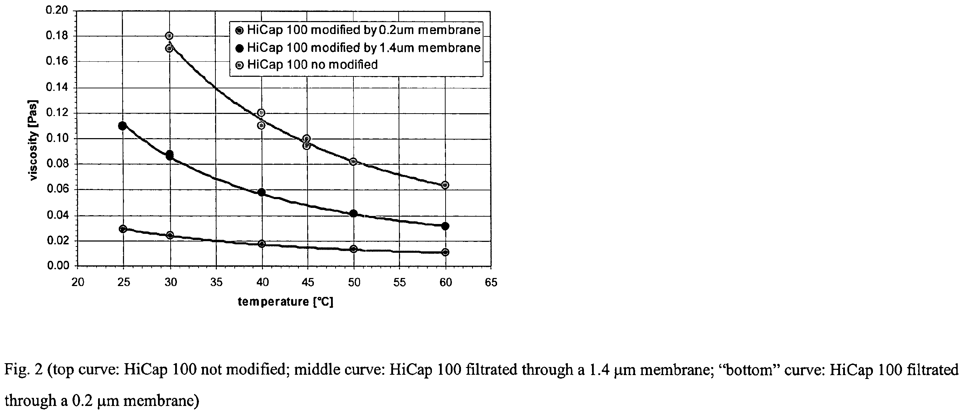

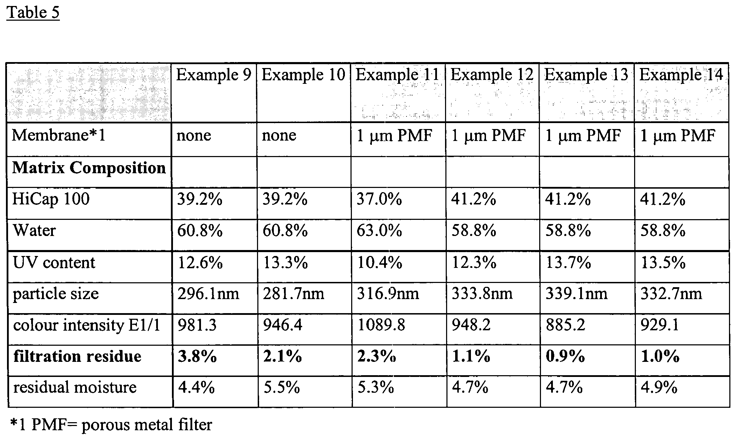

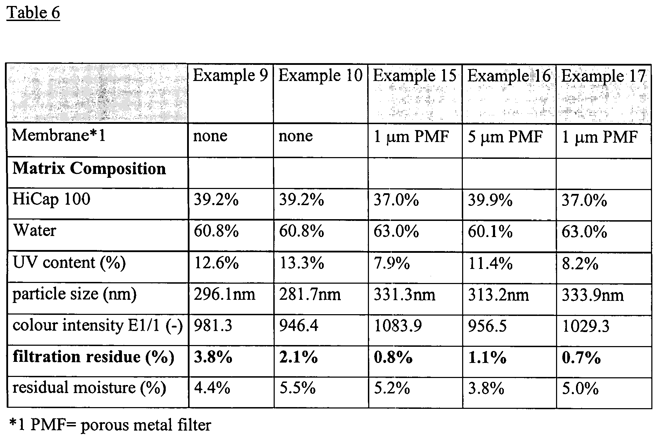

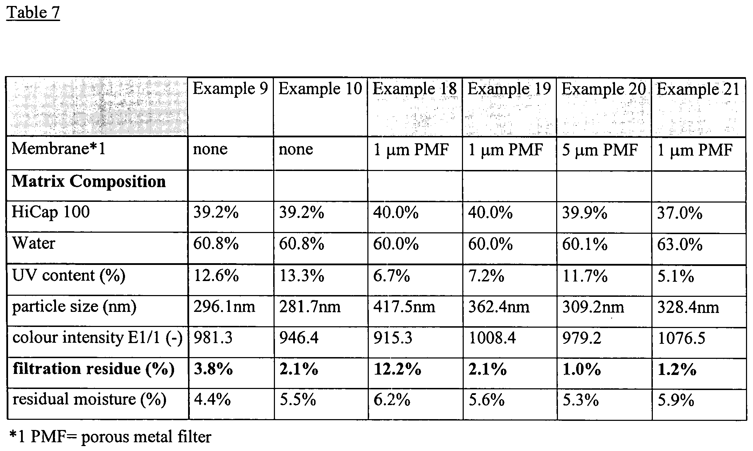

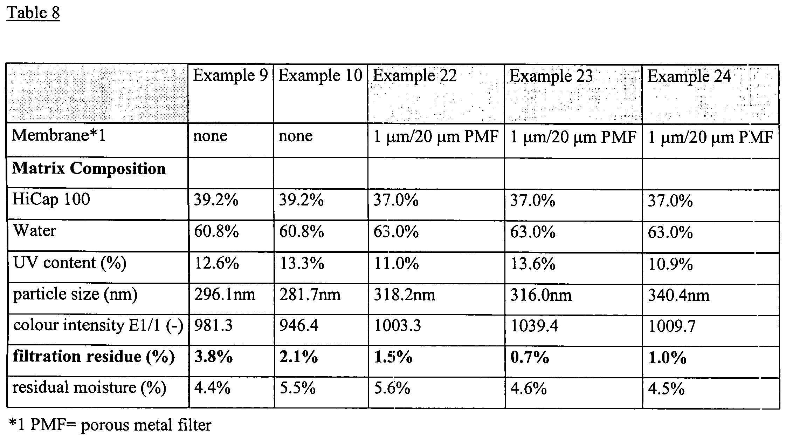

Figure 2 shows the viscosity of the filtrated starch solution (starch concentration 43 weight-%).

### Example 3: Emulsification trials

Compositions according to the present invention were manufactured according to the following procedure:
β-Carotene was dissolved in an organic solvent at 56°C. The resulting solution was added to the aqueous solutions according to example 1 or 2. As comparison example an aqueous solution of HiCap 100 was used. The exact amounts of the (improved) Hi-Cap 100 and the amount of water in the emulsion (based on the total weight of the emulsion) are given in Table 2.

**Table 2**

| **ingredient** | **not improved Hi-Cap 100 (comparison example) [weight-%]** | **Hi-Cap 100 filtered through 1.4 µm ceramic membrane (example 1) [weight-%]** | **Hi-Cap 100 filtered through 0.2 µm ceramic membrane (example 2) [weight-%]** |
|---|---|---|---|
| Hi-Cap 100 | 22 | 21.4 | 21.2 |
| Water | 30.2 | 32.2 | 32.8 |

After the emulsification the solvent-free emulsion was atomised in a mixture of cornstarch and dry ice by a rotary nozzle. The resulting product was then sieved and finally dried with compressed air in a fluidized bed.

### Results:

By using the improved OSA-starches according to example 1 and 2 the emulsification process itself was more stable and less sensitive to process condition variations than by using non-improved OSA-starches.

The resulting products had either a reduced filter residue or a higher colour intensity as can be seen in Table 3. The properties of the emulsion and the final powder were also more similar to each other when filtrated HiCap 100 was used instead of unfiltrated HiCap 100.

The filtration residue is a value determining the quality of an emulsion resulting from solving the product (the composition) as manufactured in example 3 in water (as is done e.g. when the composition is used for colouring beverages) at room temperature. The filtration residue is the amount of composition (mainly free active ingredient such as β-carotene) that remains on the filter when the emulsion is filtered through a paper filter. A filtration residue below 2 weight-% is a sign for a good emulsifying capacity of the product/composition. A high filtration residue is a sign that the active ingredient was not sufficiently incorporated into the matrix of the hydrocolloid (i.e. the (improved) OSA-starch).

A higher colour intensity means that less composition/powder is needed to achieve the same colour of food, feed, beverage etc.

**Table 3: Properties of the emulsion and powder with non-filtrated and filtrated starch solutions.**

| **Analysis** | **particle size [nm]** | **colour intensity E 1/1** | **filtration residue [weight-%]** |
|---|---|---|---|
| **Emulsion of non-improved Hi-Cap 100 (comparison example)** | 418.0 | 788.6 | 2.9 |
| **Composition (according to Table 2) containing non-improved Hi-Cap 100 (comparison example)** | 430.0 | 716.3 | 2.9 |
| **Emulsion of improved Hi-Cap 100 (example 1)** | 371.8 | 606.7 | 0.6 |
| **Composition (according to Table 2)** | 372.2 | 602.9 | 0.9 |
| **containing improved Hi-Cap 100 (example 1)** | | | |
| **Emulsion of improved Hi-Cap 100 (example 2)** | 256.2 | 946.7 | 0.4 |
| **Composition (according to Table 2) containing improved Hi-Cap 100 (example 2)** | 255.6 | 908.5 | 0.7 |

### Examples 4-8: Microfiltration of an OSA-starch with ceramic and porous metal filter membranes of different pore size.

An aqueous 40 weight-% solution of HiCap 100 (commercially available from National Starch) was filtered through one of the following membranes:
- a porous metal filter with a pore size of 5 µm (example 4),
- a porous metal filter with a pore size of 1 µm (example 5),
- a porous metal filter with a pore size of 0.5 µm (example 6),
- a ceramic membrane with a pore size of 1.4 µm (example 7),
- a ceramic membrane with a pore size of 0.8 µm (example 8).

The used porous steel pipe-filters are commercially available from LIGACON W.Röll & CO. AG (Switzerland).

The resulting filtered solutions were spray-dried. The spray-dried improved Hi-Cap 100 starches were dissolved again and converted into β-carotene compositions as described in example 3. The results for the emulsion (status after step V) are shown in the following table 4.

**Table 4**

| Example | **Membrane*1** | **particle size [nm]** | **filtration residue (%)** |
|---|---|---|---|
| comparison example | none | 313.3 | **6.7%** |
| example 4 | 5 µm PMF | 335.2 | **6.4%** |
| example 5 | 1 µm PMF | 326.8 | **4.6%** |
| example 6 | 0.5 µm PMF | 334.4 | **2.6%** |
| example 7 | 1.4 µm CM | 317.2 | **1.0%** |
| example 8 | 0.8 µm CM | 326.5 | **1.0%** |

| | | | |
|---|---|---|---|
| *1 PMF= porous metal filter, CM= ceramic membrane | | | |

The comparison example is an emulsification trial (according to example 3) with non-improved Hi-Cap 100.

### Examples 9-24: Microfiltration of an OSA-starch with porous metal filter membranes of different pore size.

### Examples 9 and 10: Comparison examples

Example 9 and example 10 are comparison examples, i.e. non-improved Hi-Cap 100 was used. With this non-improved OSA-starches products were manufactured as described in example 3. The results are disclosed in Tables 5 to 8.

### Examples 11 to 14: Microfiltration of HiCap 100 with a 1 µm porous metal filter membrane

An aqueous solution of HiCap 100 (commercially available from National Starch) with the concentration as given in Table 5 was filtered through a porous metal filter with a pore size of 1 µm. The permeate was further used for the preparation of a composition as described in example 3. The results are disclosed in Table 5.

### Example 15: Microfiltration of HiCap 100 with a 1 µm porous metal filter membrane

An aqueous 37 weight-% solution of HiCap 100 (commercially available from National Starch) was filtered through a porous metal filter with a pore size of 1 µm. The permeate was further used for the preparation of a composition as described in example 3. As additional step non-filtrated (= original) HiCap 100 was added to the emulsion before performing the powder-catch step. The results are disclosed in Table 6.

### Example 16: Microfiltration of HiCap 100 with a 5 µm porous metal filter membrane

An aqueous 39.9 weight-% solution of HiCap 100 (commercially available from National Starch) was filtered through a porous metal filter with a pore size of 5 µm. The permeate was further used for the preparation of a composition as described in example 3. As additional step non-filtrated (= original) HiCap 100 was added to the emulsion before performing the powder-catch step. The results are disclosed in Table 6.

### Example 17: Microfiltration of HiCap 100 with a 1 µm/20 µm porous metal filter membrane

An aqueous 37 weight-% solution of HiCap 100 (commercially available from National Starch) was filtered through a porous metal filter with a pore size of 1 µm. The retentate was further filtered through a porous metal filter with a pore size of 20 µm and the permeate of this filtration step used for the preparation of a composition as described in example 3. As additional step non-filtrated (= original) HiCap 100 was added to the emulsion before performing the powder-catch step. The results are disclosed in Table 6.

### Example 18: Microfiltration of HiCap 100 with a 1 µm porous metal filter membrane

An aqueous 40 weight-% solution of HiCap 100 (commercially available from National Starch) was filtered through a porous metal filter with a pore size of 1 µm. The permeate was further used for the preparation of a composition as described in example 3. As additional step the retentate obtained by the filtration step was added to the emulsion during emulsification. The results are disclosed in Table 7.

### Example 19: Microfiltration of HiCap 100 with a 1 µm porous metal filter membrane

An aqueous 40 weight-% solution of HiCap 100 (commercially available from National Starch) was filtered through a porous metal filter with a pore size of 1 µm. The permeate was further used for the preparation of a composition as described in example 3. As additional step the retentate obtained by the filtration step was added to the emulsion before performing the powder-catch step. The results are disclosed in Table 7.

### Example 20: Microfiltration of HiCap 100 with a 5 µm porous metal filter membrane

An aqueous 39.9 weight-% solution of HiCap 100 (commercially available from National Starch) was filtered through a porous metal filter with a pore size of 5 µm. The permeate was further used for the preparation of a composition as described in example 3. As additional step the retentate obtained by the filtration step was added to the emulsion before performing the powder-catch step. The results are disclosed in Table 7.

### Example 21: Microfiltration of HiCap 100 with a 1 µm porous metal filter membrane

An aqueous 37 weight-% solution of HiCap 100 (commercially available from National Starch) was filtered through a porous metal filter with a pore size of 1 µm. The permeate was further used for the preparation of a composition as described in example 3. The retentate of this filtration step was further filtered through a porous metal filter with a pore size of 20 µm. The permeate of this 20 µm filtration step was added to the emulsion before performing the powder-catch step. The results are disclosed in Table 7.

### Example 15*R: Microfiltration of HiCap 100 with a 1 µm porous metal filter membrane and use of the retentate

An aqueous solution of HiCap 100 (commercially available from National Starch) was filtered through a porous metal filter with a pore size of 1 µm. The retentate was further used for the preparation of a composition as described in example 3.

### Examples 22-24: Microfiltration of HiCap 100 with a 1 µm/20 µm porous metal filter membrane

An aqueous 37 weight-% solution of HiCap 100 (commercially available from National Starch) was filtered through a porous metal filter with a pore size of 1 µm. The retentate of this 1 µm filtration step was further filtered through a porous metal filter with a pore size of 20 µm. The permeate of this 20 µm filtration step was further used for the preparation of a composition as described in example 3. The results are disclosed in Table 8.

### Example 25: Microfiltration by use of a Hydrosart 0.45 membrane

An aqueous 30 weight-% solution of HiCap 100 (commercially available from National Starch) was filtered through a Hydrosart 0.45 membrane (commercially available from Sartorius).

### Example 26: Separation by centrifugation

An aqueous 20 weight-% solution/suspension of Hi-Cap 100 (commercially available from National Starch) was kept for 2 hours at 60°C under stirring and additionally kept without heating for 12 hours (end temperature: 40°C). The suspension was clarified using a disk separator type SC 20-06-076 of Westfalia AG (m² of separating disk package = 26000) applying 7650 rpm, equivalent to 8500 g, a volume flow of 500 1/h and 4 bar counter pressure. The clarified solution of the modified food starch (=improved modified food starch I) was spray dried.

### Example 27: Emulsification trials

A composition according to the present invention was manufactured according to the following procedure:
A) The spray-dried Hi-Cap 100 according to example 26 was dissolved in water. The suspension was then heated up to ca. 40°C and stirred for 60 minutes at 1000 rotations per minute using a dissolver disk. The suspension was kept at ca. 40°C and a pH of ca. 4 for 10 minutes.
B) β-Carotene, dl-α-tocopherol and corn oil were dissolved in an organic solvent and stirred at 70°C at 500 rotations per minute for 30 minutes using a dissolver disk.

The resulting solution B was added to the aqueous solution A under stirring at 5600 rotations per minute and kept for 30 minutes at ca. 50°C at 5000 rotations per minute using a dissolver disk. The organic solvent was removed during 60 minutes at a rotator evaporator at 55°C, 20 rotations per minute and at a final pressure of ca. 170 mbar (absolute). The foamy emulsion was centrifuged at 50°C for 10 minutes at 3000 rotations per minute (≈ 1700 g). Afterwards it was sprayed into a cooled, fluidized bed of corn starch. Further corn starch was added and the obtained beadlets were kept in the bed for 30 minutes until a temperature of 15°C was achieved. The superfluous corn starch was removed and the beadlets were dried in stream of air for 2 hours.

As comparison example (example 28) an aqueous solution of HiCap 100 was used. The average amounts of the ingredients are given in Table 9.

**Table 9:**

| **ingredient** | **amount of ingredient** | **amount of ingredient [%]** |
|---|---|---|
| β-carotene | 20.4 g | 11.5 |
| corn oil | 9.7 g | 5.5 |
| dl-α-tocopherol | 2.7 g | 1.5 |
| organic solvent (removed later) | 255 ml | - |
| improved Hi-Cap 100 according to example 26 | 100 g | 56.5 |
| Corn starch (calculated) | 35 g | 20 |
| Water (partly removed later) | 30.2 | 5 |
| **Total** | | **100** |

### Results:

The results are summarized in table 10.

**Table 10: Properties of beadlets with unchanged, clarified (via disk separation) and filtrated (via disk separation and diafiltration) starch solutions.**

| **Analysis** | **Mean particle size [nm]** | **colour intensity** | **filtration residue [weight-%]** |
|---|---|---|---|
| **Composition (according to Table 9) containing non-improved Hi-Cap 100 (comparison example 27)** | 307 nm | 744 at 477 nm | 7.2 |
| **Composition (according to Table 9) containing improved Hi-Cap 100 according to example 26** | 298 nm | 715 at 477 nm | 2.5 |

The filtration residue is a value determining the quality of an emulsion resulting from solving the product (the composition) as manufactured in example 27 in water (as is done e.g. when the composition is used for colouring beverages) at room temperature. The filtration residue is the amount of composition (mainly free active ingredient such as β-carotene) that remains on the filter when the emulsion is filtered through a paper filter. A low filtration residue is a sign for a good emulsifying capacity of the product/composition. A high filtration residue is a sign that the active ingredient was not sufficiently incorporated into the matrix of the hydrocolloid (i.e. the (improved) modified polysaccharide).

A higher colour intensity (product form dispersed in water; measured at λ(Eₘₐₓ); baseline correction at 650 nm (20 °C) at 20°C in water at the wavelength (λ) showing maximal absorption (baseline correction at 650 nm)) means that less composition/powder is needed to achieve the same colour of food, feed, beverage etc.

### Example 29

In addition to example 26, any other starch, e.g. Capsul HS (commercially available from National Starch) could be used and subjected to clarification via disk separation and optional diafiltration (diafiltration is an ultrafiltration where the permeate is substituted with H₂O).

Furthermore, mixtures (ratios of from 1:99 up to 99:1, preferred 50:50) of these improved modified food starches are suitable for use in compositions according to example 27.

A composition (example 29) has been prepared using a mixture (ratio 50:50) of improved Hi Cap 100 and improved Capsul HS (both produced analogously to example 26).

As comparison example (example 30) an aqueous solution of a mixture of HiCap 100 and Capsul HS (ratio 50 : 50) was used. The average amounts of the ingredients are given in detail in Table 9.

### Results:

The results are summarized in table 11.

**Table 11: Properties of beadlets with unchanged and clarified (via disk separation) starch solutions.**

| **Analysis** | **particle size [nm]** | **colour intensity** | **filtration residue [weight-%]** |
|---|---|---|---|
| **Composition (according to Table 9) containing a mixture of non-improved Hi-Cap 100 and non-improved Capsul HS ratio 50:50 (comparison example 30)** | 312 nm | 859 at 477 nm | 2.1 |
| **Composition (according to Table 9) con-taining a mixture of improved Hi-Cap 100 and improved Capsul HS ratio 50:50 (example 29)** | 340 nm | 846 at 477 nm | 2.8 |

### Example 31: Separation by centrifugation

An aqueous 38 weight-% solution/suspension of Hi-Cap 100 (commercially available from National Starch) was kept for 2 hours at 50°C under stirring and subsequently cooled to room temperature. The suspension was kept at room temperature for several hours, heated to 65°C and clarified using a disk separator type CSA 160-47-076 of Westfalia AG ("Quadratmeter des Tellerpaketes" = 160000 m²) applying 6.800 rpm (≈ 15000 g), a volume flow of ca. 500 1/h or higher and 6-9 bar counter pressure. The clarified solution of the modified food starch (=improved modified food starch) can be used for formulation of active ingredients, e.g. β-carotene according to example 26.

### Example 32: Separation by centrifugation

An aqueous 20 weight-% solution/suspension of Hi-Cap 100 (commercially available from National Starch) was heated for 2 hours at 60°C under stirring and additionally kept without heating for 12 hours (end temperature: 40°C). The suspension was clarified using a disk separator type SC 35 of Westfalia AG ("Quadratmeter des Tellerpaketes" = 48000 m²) applying 7250 rpm (≈ 7500 g), a volume flow of ∼750 kg/h and 6-8 bar counter pressure. The clarified solution of the modified food starch (=improved modified food starch) was spray dried.

### Example 33: Emulsification trial

A composition according to the present invention was manufactured according to the following procedure:
A) The spray-dried Hi-Cap 100 according to example 32 was dissolved in water. The suspension was then heated up to ca. 40°C and stirred for 20 min at 1000 rotations per minute using a dissolver disk. The suspension was then heated to ca. 50°C and kept at a pH of ∼4 for 10 minutes.
B) β-Carotene, dl-α-tocopherol and corn oil were dissolved in an organic solvent and stirred at 70°C at 500 rotations per minute for 30 minutes using a dissolver disk.

The resulting solution B was added to the aqueous solution A under stirring at 5600 rotations per minute and kept for 30 minutes at ca. 50°C at 5000 rotations per minute using a dissolver disk. The organic solvent was removed during 30 minutes at a rotator evaporator at 55°C, 20 rotations per minute and at a final pressure of ca. 170 mbar (absolute). The foamy emulsion was centrifuged at 50°C for 10 minutes at 3000 rotations per minute (≈ 1700 g). Afterwards it was sprayed into a cooled, fluidized bed of corn starch. Further corn starch was added and the obtained beadlets were kept in the bed for 30 minutes until a temperature of 15°C was achieved. The superfluous corn starch was removed and the beadlets were dried in stream of air for 2 hours.

As comparison example (example 34) an aqueous solution of HiCap 100 was used. The exact amounts of the ingredients are given in Table 12.

**Table 12:**

| **ingredient** | **amount of ingredient** | **amount of ingredient [%]** |
|---|---|---|
| β-carotene | 20.4 g | 11.5 |
| corn oil | 9.7 g | 5.5 |
| dl-α-tocopherol | 2.7 g | 1.5 |
| organic solvent | 215 ml | - |
| improved Hi-Cap 100 according to example 32 | 100 g | 56.5 |
| Corn starch | 35 | 20 |
| Water | 30.2 | 5 |
| **Total** | - | **100** |

### Results:

The results are summarized in table 13.

**Table 13: Properties of the emulsion and beadlets with unchanged and ultrafiltrated starch solutions.**

| **Analysis** | **particle size [nm]** | **colour intensity** | **filtration residue [weight-%]** |
|---|---|---|---|
| **Composition (according to Table 12) containing non-improved Hi-Cap 100 (comparison example 34)** | 307 nm | 744 at 477 nm | 7.2 |
| **Composition (according to Table 12) containing improved Hi-Cap 100 (example 33)** | 323 nm | 823 at 477 nm | 5.0 |

The filtration residue is a value determining the quality of an emulsion resulting from solving the product (the composition) as manufactured in example 33 in water (as is done e.g. when the composition is used for colouring beverages) at room temperature. The filtration residue is the amount of composition (mainly free active ingredient such as β-carotene) that remains on the filter when the emulsion is filtered through a paper filter. A low filtration residue is a sign for a good emulsifying capacity of the product/composition. A high filtration residue is a sign that the active ingredient was not sufficiently incorporated into the matrix of the hydrocolloid (i.e. the (improved) modified polysaccharide).

A higher colour intensity (product form dispersed in water; measured at λ(Eₘₐₓ); baseline correction at 650 nm (20 °C) at 20°C in water at the wavelength (λ) showing maximal absorption (baseline correction at 650 nm)) means that less composition/powder is needed to achieve the same colour of food, feed, beverage etc.

### Example 35: Measurement of the turbidity

As a measure of the degree of purification (separation of insoluble parts from aqueous solutions) of centrifuged OSA-starches, values for turbidity of defined solutions are suitable. The turbidity of said aqueous solutions is measured spectrophotometrically at a wavelength of 455 nm using a HACH 2100 AN Turbidimeter according to USEPA Method 180.1 at room temperature and at atmospheric pressure. The turbidity is then expressed in nephelometric turbidity units (NTU).

**Table 14 illustrates the turbidity of several purified (=improved) modified food starches using disk separation technology compared to non-improved material.**

| **Food Starch modified** | **Parameter separation: Separator type, centrifugal force, temperature, counter pressure, preferred flow rate** | **Dry matter in aqueous solution [weight-%]** | **Turbidity [NTU]** | **Turbidity** / **dry matter adjusted to 10 weight % [NTU]** |
|---|---|---|---|---|
| Cerestar C*EmCap 12635 prior separation (1) | - | 30 | - | 245 |
| Cerestar C*EmCap 12635 past separation (2) | Westfalia SC 20 7500 rpm, ∼30°C, 4 bar, 500 l/h | 30 | - | 101 |
| Capsul HS prior separation (1) | - | 30 | - | 230 |
| Improved Capsul HS past separation (2) | Westfalia SC 20 7500 rpm, ∼30°C, 4 bar, 500 l/h | 30 | - | 40 |
| Hi Cap 100 prior separation | - | 20 | 1020 | 495 |
| (1) | | | | |
| Improved Hi Cap 100 past separation (2) | Westfalia SC 20 7500 rpm, ∼40°C, 4 bar, 500 l/h | 20 | 132 | 88 |
| Hi Cap 100 Prior separation (1) | - | 35 | 1373 | 555 |
| Improved Hi Cap 100 Past separation (2) | Westfalia CSA 160-47-076, 6800 rpm, 70°C, 6-9 bar, 600 kg/h | 35 | 92 | 54 |
| Hi Cap 100 Trial code UT 06060007 Prior separation (1) | - | 20 | - | 617 |
| Improved Hi Cap 100 Past separation (2) | Westfalia SC 35, 7500 rpm, ∼75°C, 750 kg/h 8,5 bar | 20 | - | 100 |

| | | | | |
|---|---|---|---|---|
| (1) = comparison example; (2) = example according to the invention | | | | |

Decreased values for turbidity indicate improvement of the raw materials (i.e. "prior separation") used regarding separation of non-soluble parts.

### Example 36: Measurement of E1/1

An adequate amount of the formulation is dispersed, dissolved and/or diluted in/with water by use of ultrasconics in a water bath of 50 to 55°C. The resulting "solution" is diluted to a final concentration of the fat-soluble active ingredient of 10 ppm and its UV/VIS-spectrum is measured against water as reference. From the resulting UV/VIS spectrum the absorbance at the specified wavelength of maximum or shoulder, Amax, is determined. Furthermore, the absorbance at 650 nm, A650, is determined. The color intensity E1/1 is the absorbance of a 1% solution and a thickness of 1 cm and is calculated as follows: E1/1 = (Amax-A650)*dilution factor / (weight of sample * content of product form in %).

## Claims

1. Process for the manufacture of an improved modified polysaccharide comprising the following steps:
a) preparing an aqueous solution or suspension of a modified polysaccharide having a dry mass content in the range of from 0.5 to 80 weight-%, based on the total weight of the aqueous solution or suspension, whereby the temperature of the water is preferably in the range of from 1 to < 100°C;
b) separating parts of the modified polysaccharide in water at a temperature in the range of from 1 to < 100°C,
c) optionally converting the thus obtained improved modified polysaccharide into a solid form;
whereas step b) is performed by sedimentation/centrifugation and microfiltration and the dry mass content of the aqueous solution or suspension of the modified polysaccharide in step a) is in the range of from 0.5 to 80 weight-%, and
wherein the modified polysaccharide is an OSA-starch.

2. The process according to claim 1, wherein in step a) the water has a temperature in the range of from 30 to 75°C.

3. The process according to claim 1 or 2, wherein in step b) the sedimentation / centrifugation is first carried out followed by the filtration.

4. The process according to one or more of claims 1 to 3, wherein the temperature at which step b) is carried out is in the range of from 1 to < 100°C, preferably in the range of from 30 to 70°C.

5. A composition comprising
i) at least an improved modified polysaccharide, obtained according to the process of any one of claims 1 to 4;
ii) at least a fat-soluble active ingredient and/or a colorant, and
iii) optionally at least an adjuvant and/or an excipient.

6. The composition according to claim 5, wherein the improved modified polysaccharide is a modified polysaccharide whose 10% aqueous solution has a turbidity in the range of from 1 to 200 NTU, preferably in the range of from 1 to 150 NTU, more preferably in the range of from 1 to 110 NTU, most preferably in the range of from 1 to 100 NTU.

7. The composition according to any one of claims 5 to 6, wherein the fat-soluble active ingredient and/or colorant ii) is a carotene or a structurally related polyene compound, a fat soluble vitamin, a triglyceride rich in polyunsaturated fatty acids, an oil soluble UV-A filter, an UV-B filter or a mixture thereof.

8. The composition as in claim 7, wherein the carotene or structurally related polyene compound is a carotenoid, in particular β-carotene.

9. The composition as in claim 7, wherein the fat-soluble vitamin is Vitamin A or E.

10. The composition as in any one of claims 5 to 9, wherein a co-emulgator selected from the group consisting of mono- and diglycerides of fatty acids, polyglycerol esters of fatty acids, lecithins, and sorbitan monostearate is additionally present.

11. A process for the manufacture of a composition as claimed in any one of claims 5 to 10 which comprises the following steps:
I) preparing an aqueous solution or colloidal solution of a modified polysaccharide at a temperature in the range of from 1 to < 100°C,
II) separating parts of that aqueous solution or colloidal solution obtained in step I) to obtain an aqueous solution of an improved modified polysaccharide,
III) optionally adding at least a water-soluble excipient and/or adjuvant to the solution prepared in step I) or II),
IV) preparing a solution or dispersion of at least an active ingredient, preferably of at least a fat-soluble active ingredient, and/or colorant and optionally at least a fat-soluble adjuvant and/or excipient,
V) mixing the solutions prepared in step II) to IV) with each other,
VI) homogenising the thus resulting mixture,
VII) optionally converting the dispersion obtained in step VI) into a powder, whereby optionally the parts separated in step II) (or step b)) are added partly or completely during or before the conversion, optionally under addition of water, and
VIII) optionally drying the powder obtained in step VII),
wherein the modified polysaccharide is an OSA-starch.

12. The process according to claim 11, wherein steps I) and II) are carried out as follows: the aqueous solution or suspension of the modified polysaccharide is heated up to a temperature of from > 30 to< 100°C (step I), then it is cooled down to a temperature of below 30°C, and sedimentated, microfiltered and/or ultrafiltered at this lower temperature (step II).

13. Use of a composition as claimed in any one of claims 5 to 10 for the enrichment, fortification and/or coloration of food, beverages, animal feed, cosmetics or pharmaceutical compositions.

14. Food, beverages, animal feed, cosmetics and pharmaceutical compositions containing a composition as claimed in any one of claims 5 to 10.

## Patentansprüche

1. Verfahren zur Herstellung eines verbesserten modifizierten Polysaccharids, umfassend die folgenden Schritte:
a) Herstellen einer wässrigen Lösung oder Suspension eines modifizierten Polysaccharids mit einem Trockenmassegehalt im Bereich von 0,5 bis 80 Gew.-% in Bezug auf das Gesamtgewicht der wässrigen Lösung oder Suspension, wobei die Temperatur des Wassers vorzugsweise im Bereich von 1 bis < 100°C liegt;
b) Abtrennen von Teilen des modifizierten Polysaccharids in Wasser bei einer Temperatur im Bereich von 1 bis < 100°C,
c) gegebenenfalls Umwandeln des so erhaltenen verbesserten modifizierten Polysaccharids in eine feste Form;
wobei Schritt b) durch Sedimentation/Zentrifugation und Mikrofiltration durchgeführt wird und der Trockenmassegehalt der wässrigen Lösung oder Suspension des modifizierten Polysaccharids in Schritt a) im Bereich von 0,5 bis 80 Gew.-% liegt, und
wobei es sich bei dem modifizierten Polysaccharid um eine OSA-Stärke handelt.

2. Verfahren nach Anspruch 1, wobei in Schritt a) das Wasser eine Temperatur im Bereich von 30 bis 75°C aufweist.

3. Verfahren nach Anspruch 1 oder 2, wobei in Schritt b) die Sedimentation/Zentrifugation zuerst durchgeführt wird und sich die Filtration dann anschließt.

4. Verfahren nach einem oder mehreren der Ansprüche 1 bis 3, wobei die Temperatur, bei der Schritt b) durchgeführt wird, im Bereich von 1 bis < 100°C, vorzugsweise im Bereich von 30 bis 70°C, liegt.

5. Zusammensetzung, umfassend
i) mindestens ein verbessertes modifiziertes Polysaccharid, erhalten nach dem Verfahren nach einem der Ansprüche 1 bis 4;
ii) mindestens einen fettlöslichen Wirkstoff und/oder einen Farbstoff, und
iii) gegebenenfalls mindestens ein Adjuvans und/oder einen Grundstoff.

6. Zusammensetzung nach Anspruch 5, wobei es sich bei dem verbesserten modifizierten Polysaccharid um ein modifiziertes Polysaccharid handelt, dessen 10%ige wässrige Lösung eine Trübheit im Bereich von 1 bis 200 NTU, vorzugsweise im Bereich von 1 bis 150 NTU, stärker bevorzugt im Bereich von 1 bis 110 NTU, am stärksten bevorzugt im Bereich von 1 bis 100 NTU, aufweist.

7. Zusammensetzung nach einem der Ansprüche 5 bis 6, wobei es sich bei dem fettlöslichen Wirkstoff und/oder Farbstoff ii) um ein Carotin oder eine strukturverwandte Polyenverbindung, ein fettlösliches Vitamin, ein an mehrfach ungesättigten Fettsäuren reiches Triglycerid, einen öllöslichen UV-A-Filter, einen UV-B-Filter oder eine Mischung davon handelt.

8. Zusammensetzung nach Anspruch 7, wobei es sich bei dem Carotin oder der strukturverwandten Polyenverbindung um ein Carotenoid, insbesondere β-Carotin, handelt.

9. Zusammensetzung nach Anspruch 7, wobei es sich bei dem fettlöslichen Vitamin um Vitamin A oder E handelt.

10. Zusammensetzung nach einem der Ansprüche 5 bis 9, wobei zusätzlich ein Hilfsemulgator, ausgewählt aus der Gruppe bestehend aus Mono- und Diglyceriden von Fettsäuren, Polyglycerinestern von Fettsäuren, Lecithinen und Sorbitanmonostearat vorliegt.

11. Verfahren zur Herstellung einer Zusammensetzung nach einem der Ansprüche 5 bis 10, das die folgenden Schritte umfasst:
I) Herstellen einer wässrigen Lösung oder kolloidalen Lösung eines modifizierten Polysaccharids bei einer Temperatur im Bereich von 1 bis <100°C,
II) Abtrennen von Teilen der in Schritt I) erhaltenen wässrigen Lösung oder kolloidalen Lösung, wodurch man zu einer wässrigen Lösung eines verbesserten modifizierten Polysaccharids gelangt,
III) gegebenenfalls Zugeben von mindestens einem wasserlöslichen Grundstoff und/oder Hilfsstoff zu der in Schritt I) oder II) hergestellten Lösung,
IV) Herstellen einer Lösung oder Dispersion von mindestens einem Wirkstoff, vorzugsweise von mindestens einem fettlöslichen Wirkstoff, und/oder einem Farbstoff und gegebenenfalls mindestens einem fettlöslichen Hilfsstoff und/oder Grundstoff,
V) Mischen der in Schritt II) bis IV) hergestellten Lösungen miteinander,
VI) Homogenisieren der so entstandenen Mischung,
VII) gegebenenfalls Umwandeln der in Schritt VI) erhaltenen Dispersion in ein Pulver, wobei gegebenenfalls die in Schritt II) (oder Schritt b)) abgetrennten Teile teilweise oder vollständig während oder vor dem Umwandeln zugegeben werden, gegebenenfalls unter Zugabe von Wasser, und
VIII) gegebenenfalls Trocknen des in Schritt VII) erhaltenen Pulvers,
wobei es sich bei dem modifizierten Polysaccharid um eine OSA-Stärke handelt.

12. Verfahren nach Anspruch 11, wobei die Schritte I) und II) folgendermaßen durchgeführt werden:
die wässrige Lösung oder Suspension des modifizierten Polysaccharids wird auf eine Temperatur von >30 bis <100°C erhitzt (Schritt I), dann auf eine Temperatur von unterhalb 30°C abgekühlt und bei dieser niedrigeren Temperatur sedimentiert, mikrofiltriert und/oder ultrafiltriert (Schritt II).

13. Verwendung einer Zusammensetzung nach einem der Ansprüche 5 bis 10 für die Anreicherung, Fortifizierung und/oder Färbung von Nahrungsmittel, Getränken, Tierfutter, Kosmetika oder pharmazeutischen Zusammensetzungen.

14. Nahrungsmittel, Getränke, Tierfutter, Kosmetika und pharmazeutische Zusammensetzungen, die eine Zusammensetzung nach einem der Ansprüche 5 bis 10 enthalten.

## Revendications

1. Procédé de préparation d'un polysaccharide modifié amélioré, comprenant les étapes suivantes :
a) la préparation d'une solution ou suspension aqueuse d'un polysaccharide modifié ayant une teneur en masse sèche dans la plage allant de 0,5 à 80% en poids, sur la base du poids total de la solution ou suspension aqueuse, où la température de l'eau se trouve de préférence dans la plage allant de 1 à < 100°C ;
b) la séparation de parties du polysaccharide modifié dans l'eau à une température dans la plage allant de 1 à < 100°C ;
c) éventuellement la conversion du polysaccharide modifié amélioré ainsi obtenu en une forme solide ;
où l'étape b) est effectuée par sédimentation/ centrifugation et microfiltration et la teneur en masse sèche de la solution ou suspension aqueuse du polysaccharide modifié dans l'étape a) se trouve dans la plage allant de 0,5 à 80% en poids, et
où le polysaccharide modifié est un octénylsuccinate d'amidon sodique.

2. Procédé selon la revendication 1, dans lequel, dans l'étape a), l'eau possède une température dans la plage allant de 30 à 75°C.

3. Procédé selon la revendication 1 ou 2, dans lequel, dans l'étape b), la sédimentation/centrifugation est effectuée en premier, suivie de la filtration.

4. Procédé selon l'une ou plusieurs parmi les revendications 1 à 3, dans lequel la température à laquelle l'étape b) est effectuée se trouve dans la plage allant de 1 à < 100°C, préférablement dans la plage allant de 30 à 70°C.

5. Composition comprenant
i) au moins un polysaccharide modifié amélioré, obtenu selon le procédé selon l'une quelconque des revendications 1 à 4 ;
ii) au moins un ingrédient actif liposoluble et/ou un colorant ; et
iii) éventuellement au moins un adjuvant et/ou un excipient.

6. Composition selon la revendication 5, dans laquelle le polysaccharide modifié amélioré est un polysaccharide modifié dont une solution aqueuse à 10% possède une turbidité dans la plage allant de 1 à 200 NTU, préférablement dans la plage allant de 1 à 150 NTU, plus préférablement dans la plage allant de 1 à 110 NTU, tout préférablement dans la plage allant de 1 à 100 NTU.

7. Composition selon l'une quelconque des revendications 5 à 6, dans laquelle l'ingrédient actif liposoluble et/ou le colorant ii) est un carotène ou un composé de polyène apparenté sur le plan structurel, une vitamine liposoluble, un triglycéride riche en acides gras polyinsaturés, un filtre anti-UV A soluble dans l'huile, un filtre anti-UV B ou un mélange de ceux-ci.

8. Composition selon la revendication 7, dans laquelle le carotène ou un composé de polyène apparenté sur le plan structurel est un caroténoïde, en particulier le β-carotène.

9. Composition selon la revendication 7, dans laquelle la vitamine liposoluble est la vitamine A ou E.

10. Composition selon l'une quelconque des revendications 5 à 9, dans laquelle un co-agent émulsionnant choisi dans le groupe constitué par les mono- et diglycérides d'acides gras, les esters de polyglycérol et d'acides gras, les lécithines, et le monostéarate de sorbitane, est également présent.

11. Procédé de préparation d'une composition selon l'une quelconque des revendications 5 à 10, comprenant les étapes suivantes :
I) la préparation d'une solution aqueuse ou d'une solution colloïdale d'un polysaccharide modifié à une température dans la plage allant de 1 à < 100°C ;
II) la séparation de parties de cette solution aqueuse ou solution colloïdale obtenue dans l'étape I) afin d'obtenir une solution aqueuse d'un polysaccharide modifié amélioré ;
III) éventuellement l'addition d'au moins un excipient et/ou adjuvant hydrosoluble à la solution préparée dans l'étape I) ou II) ;
IV) la préparation d'une solution ou dispersion d'au moins un ingrédient actif, préférablement d'au moins un ingrédient actif liposoluble, et/ou d'un colorant et éventuellement d'au moins un adjuvant et/ou excipient liposoluble ;
V) le mélange des solutions préparées dans les étapes II) à IV) les unes avec les autres ;
VI) l'homogénéisation du mélange ainsi résultant ;
VII) éventuellement la conversion de la dispersion obtenue dans l'étape VI) en une poudre, où éventuellement les parties séparées dans l'étape II) (ou l'étape b)) sont ajoutées partiellement ou complètement pendant ou avant la conversion, éventuellement avec l'addition d'eau, et
VIII) éventuellement le séchage de la poudre obtenue dans l'étape VII) ;
où le polysaccharide modifié est un octénylsuccinate d'amidon sodique.

12. Procédé selon la revendication 11, dans lequel les étapes I) et II) sont effectuées comme suit : la solution ou suspension aqueuse du polysaccharide modifié est chauffée jusqu'à une température allant de > 30 à < 100°C (étape I), puis elle est refroidie jusqu'à une température inférieure à 30°C, et sédimentée, microfiltrée et/ou ultrafiltrée à cette température inférieure (étape II).

13. Utilisation d'une composition selon l'une quelconque des revendications 5 à 10, pour l'enrichissement, la fortification et/ou la coloration d'aliments, de boissons, d'aliments pour animaux, de produits cosmétiques ou de compositions pharmaceutiques.

14. Aliments, boissons, aliments pour animaux, produits cosmétiques et compositions pharmaceutiques contenant une composition selon l'une quelconque des revendications 5 à 10.
